# EUROPEAN PATENT APPLICATION

(11) **EP 3 955 256 A1**
(43) Date of publication of application: **16.02.2022**
(21) Application number: 19925504.3
(22) Date of filing: 31.12.2019
(51) Int. Cl.: G16B 40/00

(54) **NON-REDUNDANT GENE CLUSTERING METHOD AND SYSTEM, AND ELECTRONIC DEVICE**

(30) Priority: 16.04.2019 CN 201910303390
(71) Applicant: Shenzhen Institutes of Advanced Technology Chinese Academy of Sciences, Shenzhen, Guangdong 518055 (CN)
(72) Inventor: ZHENG, Zhichun, Shenzhen, Guangdong 518055 (CN); GUO, Ning, Shenzhen, Guangdong 518055 (CN); WEI, Yanjie, Shenzhen, Guangdong 518055 (CN)
(74) Representative: Lin Chien, Mon-Yin
(86) International application number: PCT/CN2019/130563
(87) International publication number: WO 2020/211466

(57) **Abstract**

A non-redundant gene set clustering method and system, as well as an electronic device are disclosed. The method includes: operation a: performing an alignment operation on an original gene set to obtain gene pairs in the original gene set that meet a similarity threshold; operation b: constructing a union-find forest based on the obtained gene pairs; operation c: obtaining gene clustering results of all classes in the original gene set based on the union-find forest; and operation d: based on the gene clustering results, selecting the longest sequence in each class as a representative sequence of the class to obtain a non-redundant reference gene set. According to this application, the non-redundant gene set clustering is performed by using BLAT alignment and based on the disjoint-set data structure, and so can take into account the similarity between more genes and improve the accuracy of de-redundancy.

## Description

### TECHNICAL FIELD

This application relates to the technical field of gene data processing, and more particularly relates to a non-redundant gene set clustering method and system, as well as an electronic device.

### BACKGROUND

With the rapid development of next-generation sequencing technology (NGS), the amount of biological sequence data has exploded. It is generally believed that if two sequences meet a certain similarity threshold condition, the two sequences are considered to be the same sequence or redundant to each other. The presence of a large number of redundant sequences will not only affect the speed of analysis of genome research, increase memory consumption, reduce the speed of the analysis process, but may also cause errors and affect the final experimental results.

Hobohm and Sander [Hobohm U, Scharf M, Schneider R, et al. Selection of representative protein data sets.[J]. Protein Science, 2010, 1(3):409-417*;* Hobohm U, Sander C. Enlarged representative set of protein structures.[J]. Protein Science, 2010, 3(3):522-524*.]* is the first clustering algorithm that accomplishes the clustering of non-redundant gene sequences. The basic idea is to first divide the collection of gene sequences into several different classes, and then find a sequence from each class to represent this class, and finally the set formed by these representative classes is the non-redundant reference gene set.

The software for removing redundancy from biological genetic data mainly includes NRDB90 [Holm L, Sander C. Removing near-neighbour redundancy from large protein sequence collections.[J]. Bioinformatics, 1998, 14(5):423-429*.],* CD -HIT[Li W, Jaroszewski L, Godzik A. Clustering of highly homologous sequences to reduce the size of large protein databases[J]. Bioinformatics, 2001, 17(3):282-283*;* Li W, Jaroszewski L, Godzik A. Tolerating some Redundancy Significantly Speeds up Clustering of Large Protein Databases[J]. Bioinformatics, 2002, 18(1):77-82*;* Li W. Fast Program for Clustering and Comparing Large Sets of Protein or Nucleotide Sequences[M]. Springer US, 2015*.*], *PICSES*[Wang G, Jr D R. PISCES: a protein sequence culling server[J]. Bioinformatics, 2003, 19(12):1589*.],* etc. They each have their own characteristics, and are composed of two parts: sequence alignment and selection of the final redundant sequence.

For the present, CD-HIT clustering is the most widely used for removing redundancy in research. CD-HIT is a software developed by the Burnham Institute in the United States to solve the problem of large-scale protein sequence redundancy. It can complete the construction of a non-redundant reference gene set in a relatively short time. The specific implementation is as follows: first sort all the sequences by their length, then start with the longest sequence to form the first sequence class, and then process the other sequences in turn such that a new sequence will be added to the sequence class if the similarity between the new sequence and the representative sequence of the existing sequence class is above the cutoff value, otherwise a new sequence class is formed.

The CD-HIT is fast mainly due to two reasons. One is the use of the word filtering method, that is, if the similarity between two sequences is 80% (assuming the sequence length is 100), then they have at least 60 identical words of length 2, at least 40 identical words of length 3, and at least 20 identical words of length 4. Based on this principle, when processing a new sequence, if the length of the identical word between the new sequence and the existing sequence cannot meet these requirements, there is no need for comparison, thus greatly reducing time consumption; another reason is the use of an index table, by which the number of identical words between sequences can be quickly calculated.

Although CD-HIT has a very high efficiency in de-redundancy and can complete the construction of a non-redundant reference gene set in a short time, it uses the new sequence to compare with the representative sequence of the current sequence class in every comparison, so that the other sequences in the current sequence class have no value as reference. For example, suppose there are three gene sequences A, B, and C that are arranged in order from largest to smallest by length, and according to the CD-HIT clustering method, A is first classified into one category, and then B and C are in turn taken out for comparison. If A and B are similar in length and they reach the threshold, while A and C do not reach the threshold, then we will get two categories AB and C. But in fact, C should also be considered a sequence similar to A. In addition, the word filter-based method leads to a limited level of redundancy that can be processed by each length of word. For example, a word of length 3 can only get sequence classes with a similarity of above 66.7%.

In view of the above problems, there is a need to provide a novel non-redundant gene set clustering method that can eliminate redundant genes as accurately as possible while improving the accuracy and efficiency in the process of gene de-redundancy.

### SUMMARY

The present application provides a non-redundant gene set clustering method and system, as well as an electronic device, which are intended to solve at least to a certain extent one of the above technical problems in the related art.

In order to solve the above problems, this application provides the following technical solutions.

There is provided a non-redundant gene set clustering method, including the following operations:
operation a: performing an alignment operation on an original gene set to obtain gene pairs in the original gene set that meet a similarity threshold;
operation b: constructing a union-find forest based on the obtained gene pairs;
operation c: obtaining gene clustering results of all classes in the original gene set based on the union-find forest; and
operation d: based on the gene clustering results, selecting the longest sequence in each class as the representative sequence of the class to obtain a non-redundant reference gene set.

The technical solution adopted in the embodiments of the application may further include: in operation a, performing the alignment operation on the original gene set may specifically include: setting a similarity threshold, and aligning the original gene set onto its own gene set through BLAT; optimizing the output information of BLAT, eliminating duplicate information and removing exactly the same sequences, and finally deleting unnecessary sequence information and retaining the sequence names of the gene pair and their respective length information.

The technical solution adopted in the embodiments of the application may further include: in operation b, constructing the union-find forest based on the obtained gene pairs may specifically include: for any two gene pairs, first using the Find operation to find the root information of the two gene pairs, and if the two gene pairs have the same root information, then use the Union operation to merge the numbers represented by the two gene pairs into a tree, and updating the root information; otherwise if the two gene pairs don't have the same root information, do not perform the Union operation.

The technical solution adopted in the embodiments of the application may further include: operation b may further include: performing path optimization on the union-find forest through a path compression operation, pointing the child nodes of each tree to the root node, and when merging trees merging the tree with a smaller number into the tree with a larger number to obtain an optimized union-find forest.

Another technical solution adopted by the embodiments of the present application is a non-redundant gene set clustering system, including:
a gene alignment module configured to perform an alignment operation on an original gene set to obtain gene pairs in the original gene set that meet a similarity threshold;
a union-find forest construction module configured to construct a union-find forest based on the obtained gene pairs;
a gene clustering module configured to obtain gene clustering results of all classes in the original gene set based on the union-find forest; and
a result output module configured to select the longest sequence in each class as the representative sequence of the class to obtain a non-redundant reference gene set based on the gene clustering results.

The technical solution adopted in the embodiments of the application may further include: in operation a, the gene alignment module performing the alignment operation on the original gene set may specifically include: setting a similarity threshold, and aligning the original gene set onto its own gene set through BLAT; optimizing the output information of BLAT, eliminating duplicate information and removing exactly the same sequences, and finally deleting unnecessary sequence information and retaining the sequence names of the gene pair and their respective length information.

The technical solution adopted in the embodiments of the application may further include: in operation b, the union-find forest construction module constructing the union-find forest based on the obtained gene pairs may specifically include: for any two gene pairs, first using the Find operation to find the root information of the two gene pairs, and if the two gene pairs have the same root information, then use the Union operation to merge the numbers represented by the two gene pairs into a tree, and updating the root information; otherwise if the two gene pairs don't have the same root information, do not perform the Union operation.

The technical solution adopted in the embodiments of the application may further include a union-find forest optimization module that performs path optimization on the union-find forest through a path compression operation, thus pointing the child nodes of each tree to the root node, and that when merging trees merges the tree with a smaller number into the tree with a larger number to obtain an optimized union-find forest.

Another technical solution adopted by the embodiments of the present application is an electronic device, including:
at least one processor; and
a memory communicatively coupled with the at least one processor;
wherein the memory stores instructions executable by the at least one processor, and the instructions when executed by the at least one processor cause the at least one processor to execute the following operations of the non-redundant gene set clustering method described:
operation a: performing an alignment operation on an original gene set to obtain gene pairs in the original gene set that meet a similarity threshold;
operation b: constructing a union-find forest based on the obtained gene pairs;
operation c: obtaining gene clustering results of all classes in the original gene set based on the union-find forest; and
operation d: based on the gene clustering results, selecting the longest sequence in each class as the representative sequence of the class to obtain a non-redundant reference gene set.

Compared with the related art, the embodiments of the present application may bring the following beneficial effects. According to the non-redundant gene set clustering method and system as well as the electronic device that are provided by the embodiments of the present application, the non-redundant gene set clustering is performed by using BLAT alignment and based on the disjoint-set data structure, and so can take into account the similarity between more genes and improve the accuracy of de-redundancy. Furthermore, based on the disjoint-set data structure, the construction of the non-redundant gene set can be completed in a very short time through further path compression optimization, thus improving the construction efficiency of non-reference gene sets.

### BRIEF DESCRIPTION OF DRAWINGS

FIG. 1 is a flowchart illustrating a non-redundant gene set clustering method according to an embodiment of the present application.
FIG. 2 is a block diagram illustrating a non-redundant gene set clustering system according to an embodiment of the present application.
FIG. 3 is a block diagram illustrating a hardware device that performs the non-redundant gene set clustering method according to an embodiment of the present application.

### DETAILED DESCRIPTION OF ILLUSTRATIVEEMBODIMENTS

For a better understanding of the objectives, technical solutions, and advantages of the present application, hereinafter the present application will be described in further detail in connection with the accompanying drawings and some illustrative embodiments. It is to be understood that the specific embodiments described here are intended for the mere purposes of illustrating this application, instead of limiting.

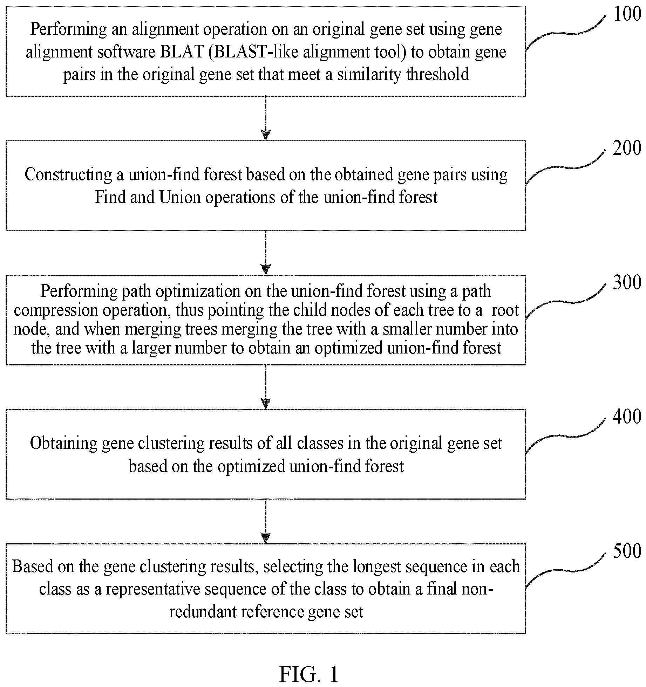

FIG. 1 is a flowchart illustrating a non-redundant gene set clustering method according to an embodiment of the present application. The non-redundant gene set clustering method according to this embodiment of the present application may include the following operations 100 to 500.

In operation 100, the method includes performing an alignment operation on an original gene set using gene alignment software BLAT to obtain gene pairs in the original gene set that meet a similarity threshold.

In operation 100, the method for obtaining the gene pair with the similarity threshold is specifically as follows: firstly set the similarity threshold, align the original gene set onto its own gene set using the gene alignment software BLAT; then optimize the output information of BLAT, where since the self-alignment is adopted, there will be situations where the sequence is aligned twice, so that in this embodiment of the present application, repeated information will be eliminated and sequences with 100% similarity (i.e., identical sequences) will be removed. Finally, some unnecessary column information is deleted, and only the sequence names of the gene pair and their respective length information are retained.

In operation b, the method includes constructing a union-find forest based on the obtained gene pairs through the Find and Union operations of the union-find forest.

In operation 200, after the alignment operation on the gene set is completed, a series of gene pairs will be obtained, and then the construction of the union-find forest may be carried out. The union-find algorithm mainly includes two operations, Find and Union. In particular,

Find: determine which subset an element belongs to, and it can be used to determine whether two elements belong to the same subset; and

Union: combine two subsets into the same set.

In this embodiment of the present application, the construction of the union-find forest is specifically as follows: for any two gene pairs, first use the Find operation to find the root information of the two gene pairs, and if the two gene pairs have the same root information, then use the Union operation to merge the numbers represented by the two gene pairs into a tree, and updating the root information. Otherwise if the two gene pairs don't have the same root information, do not perform the Union operation. As the number of gene pairs increases, the union-find forest is obtained.

In operation 300, the method includes performing path optimization on the union-find forest through a path compression operation, pointing the child nodes of each tree to the root node, and when merging trees merging the tree with a smaller number into the tree with a larger number to obtain an optimized union-find forest.

In operation 300, as the number of gene pairs increases, the height of the merged tree becomes increasingly larger, which will affect the subsequent query and merge operations. In order to solve the problem of low query efficiency caused by the excessive depth of the tree, this application uses path compression to optimize the paths of the union-find forest, which can greatly improve the clustering efficiency of non-redundant reference gene sets.

In operation 400, the method includes obtaining gene clustering results of all classes in the original gene set based on the optimized union-find forest.

In operation 500, the method includes selecting, based on the gene clustering results, the longest sequence in each class as the representative sequence of the class to obtain the final non-redundant reference gene set.

In operation 500, after the construction of the union-find forest, all the classes categorized based on clustering of the original gene set clustering are obtained. Using the stored length information, the longest sequence in each class is selected as the representative sequence to form the final non-redundant reference gene set.

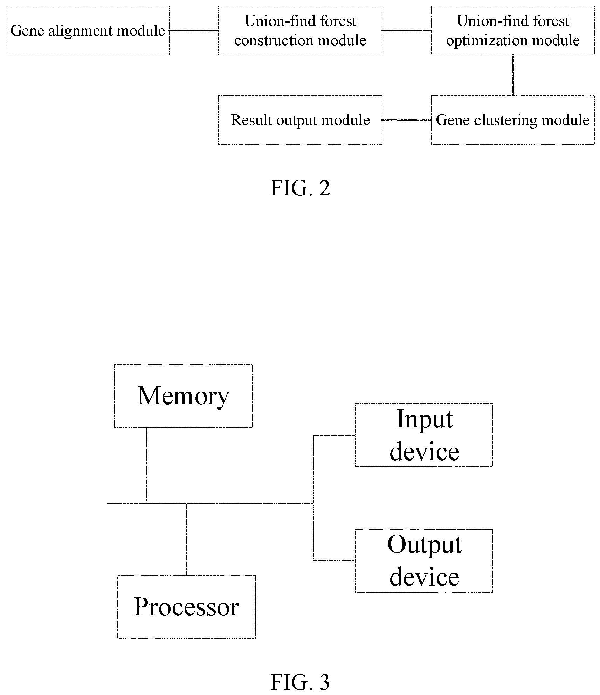

FIG. 2 is a block diagram illustrating a non-redundant gene set clustering system according to an embodiment of the present application. The non-redundant gene set clustering system according to this embodiment of the present application includes a gene alignment module, a union-find forest construction module, a union-find forest optimization module, a gene clustering module, and a result output module.

The gene alignment module is configured to perform an alignment operation on the original gene set using the gene alignment software BLAT to obtain the gene pairs in the original gene set that meet the similarity threshold. The method for obtaining the gene pair with the similarity threshold is specifically as follows: firstly set the similarity threshold, align the original gene set onto its own gene set using the gene alignment software BLAT; then optimize the output information of BLAT, where since the self-alignment is adopted, there will be situations where the sequence is aligned twice, so that in this embodiment of the present application, repeated information will be eliminated and sequences with 100% similarity (i.e., identical sequences) will be removed. Finally, some unnecessary column information is deleted, and only the sequence names of the gene pair and their respective length information are retained.

The union-find forest construction module is used to construct and optimize the union-find forest based on the obtained gene pairs through the Find and Union operations of the union-find forest. In particular, after the alignment operation on the gene set is completed, a series of gene pairs will be obtained, and then the construction of the union-find forest may be carried out. The union-find algorithm mainly includes two operations, Find and Union. In particular,

Find: determine which subset an element belongs to, and it can be used to determine whether two elements belong to the same subset; and

Union: combine two subsets into the same set.

In this embodiment of the present application, the construction of the union-find forest is specifically as follows: for any two gene pairs, first use the Find operation to find the root information of the two gene pairs, and if the two gene pairs have the same root information, then use the Union operation to merge the numbers represented by the two gene pairs into a tree, and updating the root information. Otherwise if the two gene pairs don't have the same root information, do not perform the Union operation. As the number of gene pairs increases, the union-find forest is obtained.

The union-find forest optimization module is configured to perform path optimization on the union-find forest through a path compression operation thus pointing the child nodes of each tree to the root node, and when merging trees merge the tree with a smaller number into the tree with a larger number to obtain an optimized union-find forest. In order to solve the problem of low query efficiency caused by the excessive depth of the tree, this application uses path compression to optimize the paths of the union-find forest, which can greatly improve the clustering efficiency of non-redundant reference gene sets.

The gene clustering module is configured to obtain gene clustering results of all classes in the original gene set based on the optimized union-find forest.

The result output module is configured to select the longest sequence in each class as the representative sequence of the class to obtain the final non-redundant reference gene set based on the gene clustering results. After the construction of the union-find forest, all the classes categorized based on clustering of the original gene set clustering are obtained. Using the stored length information, the longest sequence in each class is selected as the representative sequence to form the final non-redundant reference gene set.

FIG. 3 is a block diagram illustrating a hardware device that performs the non-redundant gene set clustering method according to an embodiment of the present application. As illustrated in FIG. 3, the device includes one or more processors and a memory. Taking one processor as an example, the device may further include an input system and an output system.

The processor, the memory, the input system, and the output system may be coupled by a bus or by other ways. In FIG. 3, the connection by a bus is illustrated as an example.

As a non-transitory computer-readable storage medium, the memory can be used to store non-transitory software programs, non-transitory computer executable programs and modules. The processor can execute various functional applications and data processing of the electronic device by running the non-transitory software programs, instructions, and modules stored in the memory, thus realizing the processing methods of the foregoing method embodiments.

The memory may include a program storage area and a data storage area, where the program storage area can store an operating system and an application program required by at least one function, while the data storage area can store data and the like. In addition, the memory may include a high-speed random access memory, and may also include a non-transitory memory, such as at least one magnetic disk storage device, a flash memory device, or other non-transitory solid-state storage devices. In some embodiments, the memory may optionally include a memory remotely arranged with respect to the processor, and these remote memories may be connected to the processing system through a network. Examples of the aforementioned network include, but are not limited to, the Internet, corporate intranets, local area networks, mobile communication networks, and combinations thereof.

The input system can receive input digital or character information, and generate a signal input. The output system may include display devices such as a display screen.

The one or more modules are stored in the memory, and when executed by the one or more processors, the following operations of any of the foregoing method embodiments are performed:
operation a: performing an alignment operation on an original gene set to obtain gene pairs in the original gene set that meet a similarity threshold;
operation b: constructing a union-find forest based on the obtained gene pairs;
operation c: obtaining gene clustering results of all classes in the original gene set based on the union-find forest; and
operation d: based on the gene clustering results, selecting the longest sequence in each class as the representative sequence of the class to obtain a non-redundant reference gene set.

The above-mentioned product can execute the methods provided in the embodiments of the present application, and have functional modules and beneficial effects corresponding to the executable methods. For technical details that are not described in detail in this embodiment, referring to the methods provided in the embodiments of this application.

Embodiments of the present application further provide a non-transitory (non-volatile) computer storage medium, which stores computer executable instructions, which can perform the following operations:
operation a: performing an alignment operation on an original gene set to obtain gene pairs in the original gene set that meet a similarity threshold;
operation b: constructing a union-find forest based on the obtained gene pairs;
operation c: obtaining gene clustering results of all classes in the original gene set based on the union-find forest; and
operation d: based on the gene clustering results, selecting the longest sequence in each class as the representative sequence of the class to obtain a non-redundant reference gene set.

Embodiments of the present application further provide a computer program product, which includes a computer program stored on a non-transitory computer-readable storage medium, the computer program includes program instructions, which when executed by a computer cause the computer to perform the following operations:
operation a: performing an alignment operation on an original gene set to obtain gene pairs in the original gene set that meet a similarity threshold;
operation b: constructing a union-find forest based on the obtained gene pairs;
operation c: obtaining gene clustering results of all classes in the original gene set based on the union-find forest; and
operation d: based on the gene clustering results, selecting the longest sequence in each class as the representative sequence of the class to obtain a non-redundant reference gene set.

According to the non-redundant gene set clustering method and system as well as the electronic device that are provided by the embodiments of the present application, the non-redundant gene set clustering is performed by using BLAT alignment and based on the disjoint-set data structure, and so can take into account the similarity between more genes and improve the accuracy of de-redundancy. Furthermore, based on the disjoint-set data structure, the construction of the non-redundant gene set can be completed in a very short time through further path compression optimization, thus improving the construction efficiency of non-reference gene sets.

The foregoing merely portrays some illustrative embodiments of the present disclosure. It should be noted that those of ordinary skill in the art will be able to make multiple improvements and modifications without departing from the principle of this disclosure, and these improvements and modifications should all be regarded as falling in the scope of protection of this disclosure.

## Claims

1. A non-redundant gene set clustering method, comprising:
operation a: performing an alignment operation on an original gene set to obtain gene pairs in the original gene set that meet a similarity threshold;
operation b: constructing a union-find forest based on the obtained gene pairs;
operation c: obtaining gene clustering results of all classes in the original gene set based on the union-find forest; and
operation d: based on the gene clustering results, selecting the longest sequence in each class as a representative sequence of the class to obtain a non-redundant reference gene set.

2. The non-redundant gene set clustering method as recited in claim 1, wherein in operation a, performing the alignment operation on the original gene set comprises: setting a similarity threshold, and aligning the original gene set onto its own gene set using BLAT; optimizing output information of BLAT, eliminating duplicate information and removing identical sequences, and finally deleting unneeded sequence information and retaining sequence names of the gene pairs and their respective length information.

3. The non-redundant gene set clustering method as recited in claim 1 or claim 2, wherein in operation b, constructing the union-find forest based on the obtained gene pairs comprises: for any two gene pairs, first using Find operation to find root information of the two gene pairs, and if the two gene pairs have the same root information, then using Union operation to merge the numbers represented by the two gene pairs into a tree and updating the root information; otherwise if the two gene pairs don't have the same root information, not performing the Union operation.

4. The non-redundant gene set clustering method as recited in claim 3, wherein operation b further comprises performing path optimization on the union-find forest using a path compression operation, pointing child nodes of each tree to a root node, and when merging trees merging a tree with a smaller number into a tree with a larger number to obtain an optimized union-find forest.

5. A non-redundant gene set clustering system, comprising:
a gene alignment module, configured to perform an alignment operation on an original gene set to obtain gene pairs in the original gene set that meet a similarity threshold;
a union-find forest construction module, configured to construct a union-find forest based on the obtained gene pairs;
a gene clustering module, configured to obtain gene clustering results of all classes in the original gene set based on the union-find forest; and
a result output module, configured to select the longest sequence in each class as the representative sequence of the class to obtain a non-redundant reference gene set based on the gene clustering results.

6. The non-redundant gene set clustering system as recited in claim 5, wherein the gene alignment module performing the alignment operation on the original gene set comprises: setting a similarity threshold, and aligning the original gene set onto its own gene set using BLAT; optimizing output information of BLAT, eliminating duplicate information and removing identical sequences, and finally deleting unnecessary sequence information and retaining sequence names of the gene pairs and their respective length information.

7. The non-redundant gene set clustering system as recited in claim 5 or claim 6, wherein the union-find forest construction module constructing the union-find forest based on the obtained gene pairs comprises: for any two gene pairs, first using Find operation to find root information of the two gene pairs, and if the two gene pairs have the same root information, then using Union operation to merge the numbers represented by the two gene pairs into a tree, and updating the root information; otherwise if the two gene pairs don't have the same root information, not performing the Union operation.

8. The non-redundant gene set clustering system as recited in claim 7, further comprising a union-find forest optimization module configured to perform path optimization on the union-find forest using a path compression operation, point the child nodes of each tree to a root node, and when merging trees merge a tree with a smaller number into a tree with a larger number to obtain an optimized union-find forest.

9. An electronic device, comprising:
at least one processor; and
a memory communicatively coupled with the at least one processor;
wherein the memory stores instructions executable by the at least one processor, and the instructions when executed by the at least one processor cause the at least one processor to execute the following operations of the non-redundant gene set clustering method as recited in any one of the foregoing claims 1 to 4:
operation a: performing an alignment operation on an original gene set to obtain gene pairs in the original gene set that meet a similarity threshold;
operation b: constructing a union-find forest based on the obtained gene pairs;
operation c: obtaining gene clustering results of all classes in the original gene set based on the union-find forest; and
operation d: based on the gene clustering results, selecting the longest sequence in each class as a representative sequence of the class to obtain a non-redundant reference gene set.
